# EUROPÄISCHE PATENTSCHRIFT

(11) **EP 0 493 760 B1**
(45) Veröffentlichungstag und Bekanntmachung des Hinweises auf die Patenterteilung: **30.08.1995**
(21) Anmeldenummer: 91121928.5
(22) Anmeldetag: 20.12.1991
(51) Int. Cl.: C07C 19/08, C07C 17/38

(54) **Verfahren zur Entfernung olefinischer Verunreinigungen aus teilhalogenierten Fluorchlorkohlenwasserstoffen**
Process for the removal of olefinic impurities from partially halogenated fluorochlorocarbons
Procédé pour l'élimination d'impuretés oléfiniques de fluorochlorocarbones partiellements halogénés

(30) Priorität: 21.12.1990 DE 4041181
(43) Veröffentlichungstag der Anmeldung: 08.07.1992
(73) Patentinhaber: HOECHST AKTIENGESELLSCHAFT, 65926 Frankfurt am Main (DE)
(72) Erfinder: Hopp, Peter, Dr., W-6238 Hofheim am Taunus (DE); Jansen, Rolf-Michael, Dr., W-6233 Kelkheim (Taunus) (DE)

(56) Entgegenhaltungen:
- EP-A- 0 357 328
- EP-A- 0 370 688
- US-A- 3 696 156
- US-A- 3 996 302

## Beschreibung

Die Erfindung bezieht sich auf ein Verfahren zur Entfernung olefinischer Verunreinigungen aus teilhalogenierten Fluorchlorkohlenwasserstoffen (H-FCKW).

H-FCKW der allgemeinen Formel C₂HₘFₚA_{q}, wobei A Chlor und/oder Brom ist und m und p jeweils die Zahlen 1 bis 5 sowie q die Zahlen 0 bis 4 bedeuten, wobei die Summe m+p+q gleich 6 ist, insbesondere die Verbindungen 1,1-Dichlor-2,2,2-trifluorethan CF₃CHCl₂ (R 123) und 1,1,1,2-Tetrafluorethan CF₃CH₂F (R 134a), sind als umweltfreundliche Ersatzstoffe für vollhalogenierte Fluorchlorkohlenwasserstoffe, die im Verdacht stehen, die obere Ozonschicht der Erdatmosphäre zu zerstören, von besonderem technischen Interesse. Bei der Synthese dieser H-FCKW entstehen als Nebenprodukte häufig toxische olefinische Verunreinigungen der allgemeinen Formel CₙHₓF_{y}A_{z}, wobei A Chlor und/oder Brom ist und n die Zahlen 2 bis 44 X und y jeweils die Zahlen 1 bis 6 sowie z die Zahlen 1 bis 4 bedeuten, wobei die Summe x+y+z gleich 2n ist, insbesondere 1,1,1,4,4,4-Hexafluor-2-chlor-buten-2 (R 1326) oder 1,1-Difluorchlorethen (R 1122), die auf chemischem oder physikalischem Weg abgetrennt werden müssen, damit die H-FCKW als Verschäumungsmittel oder in der Kältetechnik ohne Gefahr für die Umwelt eingesetzt werden können.

Nach dem Stand der Technik können Olefine aus FCKW-Gemischen durch Kontakt in der Gasphase mit Metalloxiden (EP-A1-370 688 und US 3 696 156) oder durch Photochlorierung (GB-A-1 401 541) in andere Produkte überführt werden. Weiter ist bekannt, daß man fluorierte Ethylene mit Natriumborhydrid in Diglyme mit zugefügtem Wasser, Ethanol oder tertiär-Butanol bei 0 bis 5 °C (J. Fluorine Chem. 1 (1971), 121) hydrieren kann.

Die genannten Verfahren zur Entfernung von Olefinen aus FCKW sind jedoch nicht dazu geeignet, die olefinischen Verunreinigungen vollständig aus H-FCKW zu beseitigen. Bei der destillativen Reinigung der genannten H-FCKW bleiben Reste an olefinischen Verunreinigungen im Destillat. Das in der zuletzt genannten Druckschrift beschriebene Verfahren zur Hydrierung fluorierter Ethylene mit Natriumborhydrid wird ausschließlich in einem protischen, das heißt in einem wäßrigen oder alkoholischen, Medium und bei einer Temperatur von höchstens 5 °C durchgeführt. Dieses Verfahren ist jedoch zur Lösung des vorliegenden Problems wenig geeignet, da bei Verwendung wäßriger Lösungsmittel anschließend ein zusätzlicher Trocknungsschritt nötig wäre. Alkohole scheiden deshalb als Lösungsmittel aus, weil sie mit vielen FCKW und H-FCKW Azeotrope bilden.

Die Aufgabe der vorliegenden Erfindung besteht darin, ein Verfahren zur Verfügung zu stellen, das olefinische Verunreinigungen, insbesondere die bereits genannten Verbindungen R 1326 und R 1122, aus H-FCKW vollständig entfernt und in unschädliche Produkte überführt, ohne daß dabei die H-FCKW angegriffen werden.

Die Erfindung betrifft ein Verfahren zur Entfernung von Olefinen der allgemeinen Formel CₙHₓF_{y}A_{*z*}, wobei A Chlor und/oder Brom ist und n eine Zahl von 2 bis 4, x und y jeweils eine Zahl von 1 bis 6 sowie z eine Zahl von 1 bis 4 bedeuten, wobei die Summe x+y+z gleich 2n ist, aus teilhalogenierten Fluorchlorkohlenwasserstoffen der allgemeinen Formel C₂HₘFₚA_{q}, worin A Chlor und/oder Brom ist und m und p jeweils eine Zahl von 1 bis 5 sowie q eine Zahl von 0 bis 4 bedeuten, wobei die Summe m+p+q gleich 6 ist, dadurch gekennzeichnet, daß die mit den genannten Olefinen verunreinigten teilhalogenierten Fluorchlorkohlenwasserstoffe C₂HₘFₚA_{q} in Gegenwart von sauerstoffhaltigen Phasentransferkatalysatoren mit Hydriden, komplexen Hydriden und/oder starken Basen des Na oder K umgesetzt werden. Die Umsetzung wird in Abwesenheit protischer Medien durchgeführt. Bevorzugte sauerstoffhaltige Phasentransferkatalysatoren sind Kronenether oder Polyethylenglykoldialkylether der allgemeinen Formel RO(CH₂CH₂O)ₐR', worin R und R' unabhängig voneinander Methyl, Ethyl, n-Propyl, iso-Propyl, n-Butyl, sec-Butyl, iso-Butyl oder tert-Butyl und a eine Zahl von 2 bis 10 bedeuten.
Bevorzugt unter den genannten Polyethylenglykoldialkylethern sind solche, in den R und R' unabhängig voneinander Methyl oder Ethyl sind.

Bevorzugte Kronenether sind Benzo-12-krone-4, Benzo-15-krone-5, 12-Krone-4, 15-Krone-5, Dibenzo-18-krone-6, Dibenzo-24-krone-8, Dicyclohexyl-18-krone-6, Dicyclohexyl-24-krone-8, 4'-Nitrobenzo-15-krone-5 oder N,N-Dibenzyl-4,13-diaza-18-krone-6.

Bevorzugte Kronenether sind 18-Krone- 6, Dibenzo-18-krone-6 und Dicyclohexyl-18-krone-6, insbesondere 18-Krone-6.

Für das erfindungsgemäße Verfahren können als komplexe Hydride Natriumborhydrid, Natriumcyanoborhydrid, Natriumtriacetoxyborhydrid, Natriumtriethylborhydrid, Natriumtrimethoxyborhydrid und Natrium-tri-sec-butyl-borhydrid verwendet werden.

Bevorzugte komplexe Hydride sind Natriumborhydrid und Natriumcyanoborhydrid, insbesondere Natriumborhydrid.

Bevorzugte starke Basen sind Alkoholate und Hydroxide, insbesondere Natriummethanolat, Kalium-tertiär-butanolat, Natriumhydroxid und Kaliumhydroxid.

Der sauerstoffhaltige Phasentransferkatalysator wird in einer Menge von 1 bis 20 Gew.-%, bezogen auf den eingesetzten H-FCKW, eingesetzt.

Die starke Base und/oder das komplexe Hydrid wird in einer Menge von 0,5 bis 10 Gew.-%, bezogen auf den eingesetzten H-FCKW, eingesetzt. Man kann ein komplexes Hydrid gemeinsam mit einem Hydrid als starke Base einsetzen, aber nicht gemeinsam mit einem Alkoholat oder einem Hydroxid als starke Base. Weiter kann man ein Gemisch mehrerer Alkoholate und/oder Hydroxide als starke Base einsetzen, aber nicht gemeinsam mit einem Hydrid als starke Base.

Nach der Reaktion lassen sich die H-FCKW durch fraktionierte Destillation in reiner Form erhalten, was ohne die erfindungsgemäße Umsetzung der olefinischen Verunreinigungen nicht möglich ist. Das Verfahren eignet sich besonders zur Reinigung der teilhalogenierten FCKW 1,1-Dichlor-2,2,2-trifluorethan und 1,1,1,2-Tetrafluorethan, in denen 1,1,1,4,4,4-Hexafluor-2-chlor-buten-2 und/oder 1,1-Difluorchlorethen als olefinische Verunreinigungen enthalten sind.

Das erfindungsgemäße Verfahren wird im allgemeinen bei Temperaturen von 0 bis 150 °C und Normaldruck (1 bar) durchgeführt oder im Autoklaven bei Drucken von 1 bis 80 bar, vorzugsweise 15 bis 40 bar, und Temperaturen von 0 bis 150 °C, vorzugsweise von 50 bis 100 °C.

Das Verfahren wird anhand der folgenden Beispiele näher erläutert.
Der in den Beispielen verwendete H-FCKW 1,1-Dichlor-2,2,2-trifluorethan (R 123) enthielt 1500 ppm 1,1,1,4,4,4-Hexafluor-2-chlor-buten-2 (R 1326), und der H-FCKW 1,1,1,2-Tetrafluorethan (R 134a) enthielt: 2000 ppm 1,1-Difluorchlorethen (R 1122) als olefinische Verunreinigungen. Der Gehalt an R 1326 und R 1122 wurde jeweils gaschromatographisch ermittelt.

### Beispiel 1

10 ml R 123 wurden mit 0,5 g NaBH₄ in 0,5-1,0 ml 18-Krone-6 bei 20 °C gerührt. Nach 12 Stunden wurde ein Gehalt an R 1326 von 0 ppm bestimmt.

In den folgenden Beispielen wurde in analoger Weise verfahren, wobei das komplexe Hydrid bzw. die starke Base und der Phasentransferkatalysator (PTK) variiert wurden.

| Beispiel | Komplexes Hydrid oder Base | PTK | Gehalt an R 1326 in ppm nach 12 Stunden |
|---|---|---|---|
| 2 | NaBH₄ | Diethylenglykol-dimethylether | 0 |
| 3 | NaBH₄ | Polyethylenglykol-dimethylether | 0 |
| 4 | LiH | 18-Krone-6 | 1280 |
| 5 | NaH | 18-Krone-6 | 0 |
| 6 | NaOMe | 18-Krone-6 | 230 |
| 7 | KO^{t}Bu | 18-Krone-6 | 0 |
| 8 | NaOH | 18-Krone-6 | 0 |
| 9 | KOK | 18-Krone-6 | 0 |

### Beispiel 10

26 g R 134a wurden zusammen mit 2 g NaBH₄ und 4 ml Diethylenglykoldimethylether im Autoklaven bei einer Temperatur von 110 °C und einem Druck von 5,2 bar umgesetzt. Nach 12 Stunden wurde ein Gehalt an R 1122 von 0 ppm bestimmt. In den folgenden Beispielen wurde in analoger Weise verfahren, wobei NaBH₄ durch eine starke Base ersetzt wurde:

| Beispiel | Starke Base | Gehalt an R 1122 in ppm nach 12 Stunden |
|---|---|---|
| 11 | NaH | 0 |
| 12 | KO^{t}Bu | 0 |
| 13 | NaOH | 0 |

## Patentansprüche

1. Verfahren zur Entfernung von Olefinen der allgemeinen Formel CₙHₓF_{y}A_{z}, wobei A Chlor und/oder Brom ist und n eine Zahl von 2 bis 4, x und y jeweils eine Zahl von 1 bis 6 sowie z eine Zahl von 1 bis 4 bedeuten, wobei die Summe x+y+z gleich 2n ist, aus teilhalogenierten Fluorchlorkohlenwasserstoffen der allgemeinen Formel C₂HₘFₚA_{q}, worin A Chlor und/oder Brom ist und m und p jeweils eine Zahl von 1 bis 5 sowie q eine Zahl von 0 bis 4 bedeuten, wobei die Summe m+p+q gleich 6 ist, dadurch gekennzeichnet, daß die mit den genannten Olefinen verunreinigten teilhalogenierten Fluorchlorkohlenwasserstoffe C₂HₘFₚA_{q} in Gegenwart von sauerstoffhaltigen Phasentranferkatalysatoren mit Hydriden, komplexen Hydriden und/oder starken Basen des Na oder K umgesetzt werden.

2. Verfahren nach Anspruch 1, dadurch gekennzeichnet, daß die teilhalogenierten Fluorchlorkohlenwasserstoffe Verbindungen mit den Summenformeln C₂H₂F₃Cl, C₂HF₃Cl₂ oder C₂H₂F₄ sind.

3. Verfahren nach Anspruch 1, dadurch gekennzeichnet, daß die teilhalogenierten Fluorchlorkohlenwasserstoffe 1,1-Dichlor-2,2,2-trifluorethan oder 1,1,1,2-Tetrafluorethan sind.

4. Verfahren nach mindestens einem der Ansprüche 1 bis 3, dadurch gekennzeichnet, daß die Olefine Verbindungen der Formeln C₂HF₂Cl oder C₄HF₆Cl sind.

5. Verfahren nach mindestens einem der Ansprüche 1 bis 4, dadurch gekennzeichnet, daß der sauerstoffhaltige Phasentransferkatalysator ein Kronenether ist.

6. Verfahren nach mindestens einem der Ansprüche 1 bis 4, dadurch gekennzeichnet, daß der sauerstoffhaltige Phasentransferkatalysator einer der Kronenether 18-Krone-6, Dibenzo-18-krone-6 oder Dicyclohexyl-18-krone-6 ist.

7. Verfahren nach mindestens einem der Ansprüche 1 bis 4, dadurch gekennzeichnet, daß der sauerstoffhaltige Phasentransferkatalysator ein Polyethylenglykoldialkylether mit der allgemeinen Formel RO(CH₂CH₂O)ₐ R' ist, worin R und R' unabhängig voneinander Methyl, Ethyl, n-Propyl, iso-Propyl, n-Butyl, sec-Butyl, iso-Butyl oder tert-Butyl und a eine Zahl von 2 bis 10 bedeuten.

8. Verfahren nach mindestens einem der Ansprüche 1 bis 4, dadurch gekennzeichnet, daß der sauerstoffhaltige Phasentransferkatalysator ein Polyethylenglykoldialkylether mit der allgemeinen Formel RO(CH₂CH₂O)ₐ R' ist, worin R und R' unabhängig voneinander Methyl oder Ethyl und a eine Zahl von 2 bis 10 bedeuten.

9. Verfahren nach mindestens einem der Ansprüche 1 bis 8, dadurch gekennzeichnet, daß die starke Base eine Alkoholat und/oder ein Hydroxid ist.

10. Verfahren nach mindestens einem der Ansprüche 1 bis 9, dadurch gekennzeichnet, daß die starke Base Natriummethanolat, Kalium-tertiär-butanolat, Natriumhydroxid und/oder Kaliumhydroxid ist.

11. Verfahren nach mindestens einem der Ansprüche 1 bis 8 dadurch gekennzeichnet, daß man Natriumhydrid einsetzt.

12. Verfahren nach mindestens einem der Ansprüche 1 bis 8, dadurch gekennzeichnet, daß das komplexe Hydrid Natriumborhydrid oder Natriumcyanoborhydrid ist.

## Claims

1. A process for the removal of olefins of the formula CₙHₓF_{y}A_{z}, in which A is chlorine and/or bromine and n is a number from 2 to 4, x and y are each a number from 1 to 6 and z is a number from 1 to 4, the sum of x+y+z being equal to 2n, from hydrochlorofluorocarbons of the formula C₂HₘFₚA_{q}, in which A is chlorine and/or bromine and m and p are each a number from 1 to 5 and q is a number from 0 to 4, the sum of m+p+q being equal to 6, which comprises reacting the hydrochlorofluorocarbons C₂HₘFₚA_{q}, which are contaminated with the olefins mentioned, in the presence of oxygen-containing phase-transfer catalysts with hydrides, complex hydrides and/or strong bases of Na or K.

2. The process as claimed in claim 1, wherein the hydrochlorofluorocarbons are compounds having the empirical formulae C₂K₂F₃Cl, C₂HF₃Cl₂ or C₂H₂F₄.

3. The process as claimed in claim 1, wherein the hydrochlorofluorocarbons are 1,1-dichloro-2,2,2-trifluoroethane or 1,1,1,2-tetrafluoroethane.

4. The process as claimed in at least one of claims 1 to 3, wherein the olefins are compounds of the formulae C₂HF₂Cl or C₄HF₆Cl.

5. The process as claimed in at least one of claims 1 to 4, wherein the oxygen-containing phase-transfer catalyst is a crown ether.

6. The process as claimed in at least one of claims 1 to 4, wherein the oxygen-containing phase-transfer catalyst is one of the crown ethers 18-crown-6, dibenzo-18-crown-6 or dicyclohexyl-18-crown-6.

7. The process as claimed in at least one of claims 1 to 4, wherein the oxygen-containing phase-transfer catalyst is a polyethylene glycol dialkyl ether of the formula RO(CH₂CH₂O)ₐR', in which R and R', independently of one another, are methyl, ethyl, n-propyl, iso-propyl, n-butyl, sec-butyl, iso-butyl or tert-butyl and a is a number from 2 to 10.

8. The process as claimed in at least one of claims 1 to 4, wherein the oxygen-containing phase-transfer catalyst is a polyethylene glycol dialkyl ether of the formula RO(CH₂CH₂O)₆R', in which R and R', independently of one another, are methyl or ethyl and a is a number from 2 to 10.

9. The process as claimed in at least one of claims 1 to 8, wherein the strong base is an alcoholate and/or a hydroxide.

10. The process as claimed in at least one of claims 1 to 9, wherein the strong base is sodium methoxide, potassium tert-butoxide, sodium hydroxide and/or potassium hydroxide.

11. The process as claimed in at least one of claims 1 to 8, wherein sodium hydride is used.

12. The process as claimed in at least one of claims 1 to 8, wherein the complex hydride is sodium borohydride or sodium cyanoborohydride.

## Revendications

1. Procédé d'élimination d'oléfines de formule générale CₙHₓF_{y}A_{z}, dans laquelle A est le chlore et/ou le brome, n est un nombre de 2 à 4, x et y sont chacun un nombre de 1 à 6 et z est un nombre de 1 à 4, la somme x+y+z étant égale à 2n, à partir de fluorochlorohydrocarbures partiellement halogénés de formule générale C₂HₘFₚA_{q}, dans laquelle A représente le chlore et/ou le brome, m et p représentent chacun un nombre de 1 à 5 et q est un nombre de 0 à 4, la somme m+p+q étant égale à 6, caractérisé en ce que l'on fait réagir les fluorochlorohydrocarbures partiellement halogénés C₂HₘFₚA_{q} contaminés par lesdites oléfines avec des hydrures, des hydrures complexes et/ou des bases fortes de Na ou K, en présence de catalyseurs de transfert de phases oxygénés.

2. Procédé selon la revendication 1, caractérisé en ce que les fluorochlorohydrocarbures partiellement halogénés sont des composés de formule brute C₂H₂F₃Cl, C₂HF₃Cl₂ ou C₂H₂F₄.

3. Procédé selon la revendication 1, caractérisé en ce que les fluorochlorohydrocarbures partiellement halogénés sont le 1,1-dichloro-2,2,2-trifluoréthane ou le 1,1,1,2-tétrafluoréthane.

4. Procédé selon l'une au moins des revendications 1 à 3, caractérisé en ce que les oléfines sont des composés de formule C₂HF₂Cl ou C₄HF₆ Cl.

5. Procédé selon l'une au moins des revendications 1 à 4, caractérisé en ce que le catalyseur de transfert de phases oxygéné est un éther couronne.

6. Procédé selon l'une au moins des revendications 1 à 4, caractérisé en ce que le catalyseur de transfert de phases oxygéné est l'un des éthers couronnes 18-couronne-6, dibenzo-18-couronne-6 ou dicyclohexyl-18-couronne-6.

7. Procédé selon l'une au moins des revendications 1 à 4, caractérisé en ce que le catalyseur de transfert de phases oxygéné est un éther dialkylique de polyéthylèneglycol de formule générale RO(CH₂CH₂O)ₐR', dans laquelle R et R' représentent indépendamment l'un de l'autre un groupe méthyle, éthyle, n-propyle, isopropyle, n-butyle, sec-butyle, isobutyle ou tert-butyle, et a est un nombre de 2 à 10.

8. Procédé selon l'une au moins des revendications 1 à 4, caractérisé en ce que le catalyseur de transfert de phases oxygéné est un éther dialkylique de polyéthylèneglycol de formule générale RO(CH₂CH₂O)ₐR', dans laquelle R et R' représentent indépendamment l'un de l'autre un groupe méthyle ou éthyle, et a est un nombre de 2 à 10.

9. Procédé selon l'une au moins des revendications 1 à 8, caractérisé en ce que la base forte est un alcoolate et/ou un hydroxyde.

10. Procédé selon l'une au moins des revendications 1 à 9, caractérisé en ce que la base forte est le méthylate de sodium, le tert-butylate de potassium, l'hydroxyde de sodium et/ou l'hydroxyde de potassium.

11. Procédé selon l'une au moins des revendications 1 à 8, caractérisé en ce que l'on utilise de l'hydrure de sodium.

12. Procédé selon l'une au moins des revendications 1 à 8, caractérisé en ce que l'hydrure complexe est le borohydrure de sodium ou le cyanoborohydrure de sodium.
